# EUROPEAN PATENT APPLICATION

(11) **EP 0 552 032 A2**
(43) Date of publication of application: **21.07.1993**
(21) Application number: 93300215.6
(22) Date of filing: 14.01.1993
(51) Int. Cl.: C11D 1/52, C11D 1/65, A61K 7/50

(54) **Surfactant amides and cosmetic or cleaning compositions comprising the same**

(30) Priority: 16.01.1992 GB 9200903; 17.01.1992 GB 9201058
(71) Applicant: UNILEVER PLC, London EC4P 4BQ (GB); UNILEVER N.V., NL-3013 AL Rotterdam (NL)
(72) Inventor: Naik, Appayya Raghunath, Unilever Research, Bebington, Wirral, Merseyside L63 3JW (GB)
(74) Representative: Elliott, Peter William

(57) **Abstract**

The invention relates to the preparation of amides, particularly alkanolamides an gluconamides and to cosmetic or cleaning compositions containing said amides. These are known as lather boosters but contains 1-10% of the corresponding amine. This unwanted component gives a characteristically 'fishy' amine smell to products, can lead to yellowing and can lead to undesirable bi-product formation on storage. It is desirable that these disadvantages are avoided. Our solution involves a selective reaction for amine which does not substantially effect the hydroxyl function. In one aspect of the present invention the amine is reacted with ethylene oxide in the absence of a basic catalyst to form the tertiary amine. Consequently, the present invention provides cosmetic or cleaning compositions comprising amide surfactants where the ratio of the amide to the corresponding amine is greater than 800:1 and the ratio of unethoxylated amide to the corresponding ethoxylated amide is greater than 1:1.

## Description

The invention relates to the preparation of surfactant molecules having an amide head-group and to cosmetic or cleaning compositions containing said amides.

Certain amide surfactants, more specifically the short chain alkanolamides, particularly the mono and di ethanolamides and isopropanolamides, are known as lather boosters and find general applicability in a wide range of both cosmetic and cleaning preparations.

Longer chain amides, such as the glucamides are also known as surfactant molecules and have been employed as lather boosters, although the invention will be described with particular reference to the relatively short chain alkanolamides.

It is known to prepare alkanolamides on an industrial scale by condensation reaction of fatty acids, fatty acid methyl esters or fatty triacyl-glycerides with excess alkanolamine in the presence of an alkaline catalyst such as alkali metal hydroxide or alkoxide. The reaction between the above-mentioned components is complicated by the competing reactivity of the several functional groups present.

Where ethanolamine is used as one of the starting materials, the reaction produces, as its major product, fatty monoethanolamide (HO-CH₂-CH₂-NH-CO-R) and fatty diethanolamide ((HO-CH₂-CH₂)₂-N-CO-R), in which R is usually C₇-C₁₇ alkyl.

Further information on the synthesis of fatty acid and other surfactant amides can be found in 'Nonionic Surfactants' by Martin Schick (Volume 1, Marcel Dekker Inc. 1967, LCCCN 66-22492) at pages 213-225 and 395-403.

For comparative purposes, it is noted that structure of glucamides is similar to that of the alkanolamides: a typical glucamide having the structural formula given below:

HO-CH₂-(CHOH)₄-CH₂-N(CH₃)-CO-R

where R is again C7-C17 alkyl.

Some glucamides have comparable lather boosting activity to the corresponding mono-ethanolamides: lauroyl N-methyl glucamide exhibits foam-boosting properties similar to those of lauroyl mono-ethanolamide.

Commercially available mono- and di- ethanolamides are known to contain the corresponding unreacted ethanolamines. This unwanted component gives a characteristically 'fishy' amine smell to products, can lead to yellowing and can lead to undesirable bi-product formation on storage. One particularly undesirable class of bi-product which can be formed after the storage of amine-containing products are the nitrosamine derivatives. It is desirable that these disadvantages are avoided.

The levels of mono and di ethanolamine in the corresponding commercial ethanolamides can vary. Schick, as mentioned above, cites figures (p401) of 23% and 7% for typical compositions. A set of commercial Coco-diethanolamide analyses revealed that the level of free diethanolamine varied between 3-7 wt%. Purer samples can be obtained with a free diethanolamine content of 0.5-1.3 wt%. Similar, or slightly lower levels of mono-ethanolamine are expected to be present in commercial mono-ethanolamides, since mono-ethanolamine has a lower molecular weight. In general the ratio of alkanolamine to alkanolamide is expected to be, at best, of the order of 4:200, i.e. in the range 1:200 to 14:200.

While it might be considered possible to remove unreacted amine by distillation. It is believed that this rather expensive step can itself effect the colour of the products obtained by forming yellow bi-products.

It is known to avoid the high residual levels of amine by use of an ethoxylation reaction.

Ethoxylated monoethanolamide (Aminol N [RTM]: 2-3 EO) is obtained by treatment of the fatty acid/ethanolamine reaction mixture with ethylene oxide under alkaline catalytic conditions. The products of this step contain low levels of the starting amine, and a large proportion of the ethanolamide is ethoxylated: i.e ethylene oxide reacts under alkali catalytic conditions with both the hydroxyl and the amine to form the tertiary amine and the ethoxylated amine and amide.

Performance of alkanolamides as a lather booster is better if the non-ethoxylated compound is used, and consequently the ethoxylated materials are a less preferred lather booster and have to be used in higher dosages than the known, non-ethoxylated but contaminated alkanolamide.

EP 381548 by SEPPIC, (1989) states the problem of nitrosamine precursor formation, as mentioned above, and proposes a method for obtaining amide products containing in the region of 0.5% diethanolamine. EP 381548 teaches that by use of acetic anhydride to acylate the amine, high yields of the amide can be obtained. However this approach will acylate the hydroxyl function of the amine and require further steps to remove the excess anhydride and acetic acid formed.

The outstanding technical problem is therefore to prepare surfactant amides, such as mono and di ethanolamide and the corresponding glucamides, which contain little or none of the corresponding amine without resorting to ethoxylation or other modification reactions i.e. without substantially modifying the hydroxyl function. Similar problems occur in the preparation of the alternative amides such as the glucamides, i.e. it is desirable that residual glucamine is avoided.

Our solution involves a selective reaction for amine which does not substantially effect the hydroxyl function.

In one aspect of the present invention the amine is reacted with ethylene oxide in the absence of a basic catalyst to form the tertiary amine. Alternatively an ester or lactone can be reacted with the amine in excess with or without catalyst. It is preferred not to use substantial excess reagent with a basic catalyst.

The above-mentioned routes enable the production of amides which have low levels of contaminant amine and which have avoided the requirement for ethoxylation or other substantial modification of the hydroxyl function.

Consequently, the present invention provides cosmetic or cleaning compositions comprising unethoxylated surfactant amide where the ratio of said amide to the corresponding amine is greater than 800:1 and the ratio of said amide to the corresponding ethoxylated amide is greater than 1:1.

Typically, the compositions comprise a C₂-C₄ alkanolamide where the ratio of the alkanolamide to the corresponding alkanolamine is greater than 800:1 and the ratio of unethoxylated alkanolamide to the corresponding ethoxylated alkanolamide is greater than 1:1.

The alkanolamide is preferably ethanolamide, which is the preferred lather booster in the amide series, and is preferably the mono-alkanolamide which is preferred as a lather booster over di-alkanolamide. Monoethanolamide is therefore the preferred alkanolamide.

Preferably, the amide further comprises C₁₁-C₁₃ residues. A suitable source of these residues are the fatty acids obtained from lauric acid oils, such as coconut oils and fractions thereof.

Cleaning compositions which embody the present invention further comprise actives at levels of 0.1-80wt%. More preferably the active level for non-concentrated compositions is 1-30wt%, preferably around 20wt% and for concentrated compositions 30-85wt%. The concentration of active will vary with the application of the cleaning composition: less than 1% for window cleaning solutions, 1-5wt% for cleaning floors and similar surfaces, 5-15wt% for shower gels and shampoos, 15-40wt% for dishwashing liquids. The level of alkanolamide as lather booster is preferably 0.5-20wt% on active, more preferably 1-15wt% on active, most preferably 8-12wt% on active as lower levels of alkanolamide do not tend to crystallise out on storage.

Generally, the compositions according to the present invention will comprise additional surfactant components selected from the group of anionic and nonionic surfactants and more preferably a mixture of both nonionic and anionic surfactants.

Preferably the anionic surfactant is a primary alcohol sulphate (PAS), a foaming, readilly biodegradable, surfactant.

The preferred primary alcohol sulphate comprises a mixture of materials of the general formulation:

RO- SO₃X

wherein R is a C₈ to C₁₈ primary alkyl group and X is a solubilising cation. Suitable cations include sodium, magnesium, potassium, ammonium and mixtures thereof. Particularly preferred PAS molecules are those with a major proportion of C₁₀-C₁₄ alkyl residues. These can be obtained by forming the PAS from fatty acids obtained from coconut oil although they can also be obtained from synthetic alcohol sources.

The preferred nonionic surfactant is selected from the group comprising ethoxylated alcohols of the general formula:

R₁-(OCH₂CH₂)ₘ-OH

wherein R₁ is straight or branched, C₈ to C₁₈ alkyl and the average degree of ethoxylation m is 1-14, preferably 3-8. The starting materials for the synthesis of these ethoxylated alcohols, a minor component of the surfactant system, are available from both natural and synthetic sources.

Optional ingredients include perfume, colour, optional abrasives, solvents, builders, enzymes, thickeners, antimicrobial and other hygiene agents, acids, alkalis and bleaching agents. Few compatability problems have been noted between the alkanolamides and these ingredients.

Particularly preferred as optional ingredients are solvents and emulsifiers. According to one preferred embodiment of the present invention the cleaning or cosmetic compositions further comprises partial, preferably mono-, glycerides of fatty acids having a carbon chain length in the range C₇-C₂₃, preferably C₇-C₁₇. The use of such monoglycerides is advantageous as they give a further lather boost. The monoglyceride level in the final products is preferably less than 100 Mole% on alkanolamide.

According to a further preferred embodiment of the present invention the eventual product comprises as amine-reactive solvent, preferably triacetin. Triacetin can be usefully employed in the process aspects of the present invention in quantities which yield a level of this component in the final product of less than 100 Mole% on amide.

Further solvent components can be present in products according to the invention, these are commonly selected from propylene glycol mono n-butyl ether, dipropylene glycol mono n-butyl ether, propylene glycol mono t-butyl ether, dipropylene glycol mono t-butyl ether, diethylene glycol hexyl ether, ethyl acetate, methanol, ethanol, isopropyl alcohol, ethylene glycol monobutyl ether, di-ethylene glycol monobutyl ether and mixtures thereof.

Three manufacturing processes will be described in some detail. For convenience these will be referred to as treatments 1, 2 and 3. Each treatment can be conducted on an industrial scale by reaction of any of fatty acids, fatty acid methyl esters or triacyl-glycerides with alkanolamine.

Using treatment 1, ethlyene oxide is added to the alkanolamide-forming reaction mixture in the absence of a catalyst. The reaction proceeds at 150°C for one hour. The excess, unreacted ethylene oxide is subsequently stripped out of the reaction mixture.

In alternative treatment 2, excess monglycerides is added to the alkanolamide-forming reaction mixture. The eventual product will contain some residual monoglyceride giving a further lather boost. Triacylglycerides (oils and fats) do not give this benefit as they have antilather (foam breaking) properties. Monoglycerides are environmentally friendly and are quickly broken down in the environment. Monoglyceride is preferably employed in molar excess. The use of an alkaline catalyst is optional.

Preferred monoglycerides comprise residues of C6-C18 fatty acids, preferably C12-C14 fatty acids. Monoglycerides obtained form lauric fats, preferably coconut fat is preferred. Some diglycerides may be present but it is preferred that the monoglyceride is 100-50wt% pure.

Treatment 3 employs an amine reactive solvent, preferably triacetin (glycerol tri-acetate: a non-toxic solvent) or a low molecular weight ester in the alkanolamide-forming reaction mixture. Triacetin is preferably employed in excess. The triacetin need not be pure and can contain monoor di-acetin The use of an alkaline catalyst is optional.

Treatments 2 and 3 are preferred.

Preferred catalysts are alkali metal hydroxides, preferably potassium hydroxide. Alternatively, sodium methoxide or other alkoxides may be employed.

The invention is illustrated by the following examples:

Analytically pure lauryl mono-ethanolamide (LME) was prepared by crystalisation and subsequently contaminated with 1wt% monoethanolamine to model the reaction mixture. A sample of the mixture was subjected to thin-layer chromatography and the result obtained was used as a 100% contamination value in subsequent assays. TLC was performed on a silica gel HF₂₅₄ (200 x 100mm) plate [Merck Art 5729] with ethyl acetate: methanol:dilute ammonia (approx 50:30:20 v:v:v). Visualisation used ninhydrin spray.

Treatments 1-3 as described above were performed and the residual level of mono-ethanolamine determined. The results are given in the centre column of Table 1 below, under 'residue'. The right hand column of the table lists the relative foaming performance of a mixture of 90%

Dobanol 23-1EOS and 10% reaction product. Treatments 2-3 were also performed with samples of di-ethanolamine for which results are given in Table 2 below.

As mentioned above, it is known that modification of the hydroxyl function of the amide reduces the foaming performance. Consequently both the level of residual amine and the foaming performance are reported below.

Foaming performance was assayed by means of a modified Schlachter-Dierkes test based on the principle described in Fette und Seifen 1951, 53, 207. 100ml aqueous solution of each material tested having a concentration of 0.04% active detergent in 24°H water (French hardness) at 45°C was rapidly oscillated using a vertically oscillating perforated plunger within a graduated cylinder. After the initial generation of foam, increments (0.2 g) of soil (9.5 parts commercial cooking fat (contains triglyceride which is known to function as an anti foaming component), 0.25 parts oleic acid, 0.25 parts stearic acid and 10 parts wheat starch in 120 parts water) were added at 15 second intervals (10 seconds mild agitation and 5 seconds rest) until the foam collapsed. The result was recorded as the number of soil increments (NSI). A score difference of three or less is generally regarded as insignificant. Each result was typically the average of 3-4 runs.

**TABLE 1**

| **Control:** | **% Residue** | **Foam** |
|---|---|---|
| LME/1% Mono-ethanolamine | 100 | 39 |

| **Treatments:** | | |
|---|---|---|
| 1-Ethylene oxide (150°C, 1Hr) | trace | 41 |
| 2-Glycerol Monolaurate | | |
| - without catalyst ( 80°C, 3hr) | 31 | 38 |
| - with 1% KOH (100°C, 3hr) | 15 | 40 |
| 3-Triacetin | | |
| - without catalyst ( 80°C, 3hr) | 4.6 | 37 |
| - with 1% KOH (100°C, 3hr) | 3.0 | 37 |

**TABLE 2**

| **Control:** | **% Residue** | **Foam** |
|---|---|---|
| LME/1% Di-ethanolamine | 100 | - |

| **Treatments:** | | |
|---|---|---|
| | | |
| 2-Glycerol Monolaurate | | |
| -without catalyst (120°C, 6hr) | 16 | - |
| | | |
| 3-Triacetin | | |
| - without catalyst (120°C, 6hr) | 4 | - |

From the above results it can be seen that reduction in the level of alkanolamine as compared with an equivalent to the one of the better commercial alkanolamide could be obtained without a reduction in the lathering performance In other words, products could be prepared with significantly lower levels of alkanolamine without reducing the performance as regards foaming.

## Claims

1. Cosmetic or cleaning compositions comprising unethoxylated surfactant amide where the ratio of said amide to the corresponding amine is greater than 800:1 and the ratio of said amide to the corresponding ethoxylated amide is greater than 1:1.

2. Compositions according to claim 1 comprising C₂-C₄ alkanolamide where the ratio of the alkanolamide to the corresponding alkanolamine is greater than 800:1 and the ratio of unethoxylated alkanolamide to the corresponding ethoxylated alkanolamide is greater than 1:1.

3. Composition according to claim 2 wherein the alkanolamide is ethanolamide.

4. Composition according to claim 2 or 3 wherein the alkanolamide is a mono-alkanolamide.

5. Composition according to any of the preceeding claims wherein the alkanolamide comprises C₁₁-C₁₃ residues.

6. Composition according to any of the preceeding claims comprising actives at levels of 1-80wt%.

7. Composition according to any of the preceeding claims comprising a partial glyceride.

8. Composition according to claim 7 comprising mono-, glycerides of fatty acids having a carbon chain length in the range C₉-C₂₃.

9. Composition according to any of the preceeding claims comprising an amine-reactive solvent.
